(19) **Europäisches Patentamt / European Patent Office / Office européen des brevets**

(11) **EP 3 292 862 A1**

(12) **EUROPEAN PATENT APPLICATION**

(43) Date of publication:
14.03.2018 Bulletin 2018/11

(51) Int Cl.:
*A61K 9/16* (2006.01)    *A61K 31/00* (2006.01)

(21) Application number: 16187705.5

(22) Date of filing: 07.09.2016

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **SANDOZ AG**
**4056 Basel (CH)**

(72) Inventor: **The designation of the inventor has not yet been filed**

(74) Representative: **Kluschanzoff, Harald et al**
**Sandoz International GmbH**
**Global Intellectual Property**
**Industriestrasse 25**
**83607 Holzkirchen (DE)**

(54) **OMEPRAZOLE FORMULATIONS**

(57) The present invention refers to pellets comprising pharmaceutically active ingredient and binder. Further, the invention refers to a process for preparing said pellets, to the use of said pellets for preparing a pharmaceutical composition, and to a dosage form comprising said pellets or pharmaceutical composition. Finally, the present invention refers to a dosage from for use in a method of treating a disease selected from the group consisting of gastro esophageal reflux disease, peptic ulcer disease, and Zollinger-Ellison syndrome and for prevention of upper gastrointestinal bleeding in people who are at high risk.

EP 3 292 862 A1

**Description**

Field of the invention

The present invention belongs to the field of pharmaceutical industry and relates to pellets comprising pharmaceutically active ingredient and binder. Further, the invention refers to a process for preparing said pellets, to the use of said pellets for preparing a pharmaceutical composition, and to a dosage form comprising said pellets or pharmaceutical composition. Finally, the present invention refers to a dosage from for use in a method of treating a disease selected from the group consisting of gastro esophageal reflux disease, peptic ulcer disease, and Zollinger-Ellison syndrome and for prevention of upper gastrointestinal bleeding in people who are at high risk.

Description of the background art

The racemate of (*RS*)5-methoxy-2-[[(4-methoxy-3,5-dimethyl-2-pyridinyl)methyl]sulfinyl]-1H-benzimidazole is known as omeprazole and is described for instance in EP 0,005,129. Its R-enantiomer is known as esomeprazole. Also the S-enantiomer can be provided in pure form. Furthermore, pharmaceutically acceptable salts of the aforementioned compounds are known. Omeprazole is generally known to be useful for inhibiting gastric acid secretion in mammals and man by controlling gastric acid secretion at the final step of the acid secretory pathway and can be used in the treatment of gastric and duodenal ulcers. It is an inherent property of omeprazole to be activated to the active moiety in the acid environment within the parietal cells. The activated compound interacts with the enzyme in the parietal cells, which mediates the production of hydrochloric acid in the gastric mucosa. Omeprazole contains a sulfoxide grouping, which interferes with the $H^+K^+$-ATPase in the parietal cells hitherto known are all also degraded in acid media.

Omeprazole is a proton pump inhibitor (PPI) and a potent inhibitor of gastric acidity which is widely used in the therapy of gastroesophageal reflux and peptic ulcer disease. Omeprazole is an approved medicament for short-term treatment of duodenal and gastric ulcers, for the treatment of patients with symptomatic gastroesophageal reflux disease, for the treatment of erosive esophagitis and for the long-term treatment of pathological hypersecretory conditions (e.g., Zollinger-Ellison syndrome, multiple endocrine adenomas and systemic mastocytosis).

Omeprazole is however susceptible to degradation/transformation in acid reacting and neutral media. The half-life of omeprazole in water solutions at pH values less than 4 is shorter than 10 minutes. Also at neutral pH-values the degradation process proceeds rapidly, and at pH of about 7 the half-life of omeprazole is about 14 hours, while at higher pH-values the stability in solution is much better (Pilbrant and Cederberg, Scand. J. Gastroenterology 1985; 20 (supp. 108) p. 113-120). The stability profile is similar in solid phase. The degradation of omeprazole is catalyzed by acidic reacting compounds and is stabilized in mixtures with alkaline reacting compounds. The stability of omeprazole is also affected by moisture and organic solvents. Therefore, if omeprazole is administered orally, it must be protected from the gastric juices, which are acid, so that it may reach the small intestine where it is absorbed, in unaltered state (EP 0,247,983).

In human pharmacological studies it was found that the release rate of omeprazole from a pharmaceutical dosage form can influence the total extent of absorption of omeprazole to the general circulation (Pilbrant and Cederberg, Scand. J. Gastroenterology 1985; 20 (suppl. 108) p. 113-120). A fully bioavailable dosage form of omeprazole must release the active drug rapidly in the proximal part of the gastrointestinal canal.

In order to obtain a pharmaceutical dosage form of omeprazole which prevents omeprazole from contact with acidic gastric juice after oral intake, the pharmaceutical dosage form of acid labile substances must be enteric coated. Enteric polymers do not dissolve at low pH and by this protection of the drug is ensured. After the pH increase in small intestine the polymer dissolves and releases the drug. For safety and efficacy of patients it is essential that no release of omeprazole is achieved in acid environment in order to protect degradation of omeprazole. At higher pH rapid and fast release is required in order to ensure fast absorption of omeprazole and consequently pharmacological effect.

Enteric formulation is more preferred to be in form of multi-unit dosage system due to reduced risk of dose dumping and facilitated stomach passage due to smaller size of dosage form. The most common dosage form is a capsule which is filled with approximately 1 mm big particles called pellets. Pellets are usually filled into hard gelatin capsules. One of the possibilities to produce such pellets is by applying layers of pharmaceutically active ingredient and excipients onto inert cores.

Pellets comprising inert core covered by the active layer comprising omeprazole or one of its single enantiomers, optionally mixed with alkaline reacting compounds are disclosed in several prior art documents. For example WO 93/25204 discloses a stable microgranule formulation of omeprazole comprising an inert core consisting of sugar and starch covered with an active layer constituted by omeprazole diluted in mannitol in substantially equal amounts, and an intermediate layer comprising mannitol, an outer layer formed from an enteric coating being optionally present. WO 98/052564 discloses a pharmaceutical composition which is a solid pellet comprising an inert core, a benzimidazole in or on the core, preferably present in an alkaline environment, a moisture resistant coating around the core, the moisture resistant coating comprising at least one hydrophobic material, and an enteric coating around the moisture resistant coating. WO 96/23500 discloses an oral pharmaceutical formulation containing omeprazole which comprises a nucleus formed by an inert core onto which an active layer containing omeprazole, non-alkaline reacting pharmaceutical excipient (e.g. talc) and water soluble inert polymer (e.g. hydroxypropylmethycellulose, HPMC) is sprayed.

There are two main technologies for the production of pellets comprising an inert core covered by the layer comprising pharmaceutically active ingredient. The first technology is a technology comprising wet granulation / extrusion / spheronization process, the method wherein after granulation by extrusion the inert cores are made spherical e.g. with a marumerizer. The inert cores so produced are most often not of an approximately spherical shape, and the granule size distribution is wide and therefore the uniform coating is difficult to achieve. In addition, carrying out the wet granulation / extrusion / spheronization manufacturing process normally requires the transfer of inert cores from extruder / spherionizer to the coating pans or fluid bed equipment in order to be coated. It also requires various equipment of restricted use and a high costs which is reflected in the final price of product.

The second technology is a drug layering process by using spraying of inert cores with suspension or solution comprising pharmaceutically active ingredient in fluidized bed spray granulator. The layering process usually begins with inert spherical cores that provide the solid surface on which the active layer comprising pharmaceutically active ingredient (also referred to herein as active agent, API or drug) is sprayed. Inert cores are traditionally formed from pharmaceutically acceptable substances, such as lactose, sucrose, and starch. In prior art wherein omeprazole formulations containing pellets that comprise an inert core covered by the active layer comprising omeprazole, the drug loading of less than about 15 % of omeprazole was used and the usual ratio between omeprazole and inert cores of more than 1:4 was identified. For example in WO 93/25204 the drug loading of less than 10 % of omeprazole in final pharmaceutical formulation and the ratio between omeprazole and inert cores of 1:4 is disclosed; in WO 96/23500 the drug loading of 6.6 % of omeprazole in final pharmaceutical formulation and the ratio between omeprazole and inert cores of 1:7 is disclosed; in WO 98/052564 the drug loading of 3.6 % of omeprazole in final pharmaceutical formulation and high ratio between omeprazole and inert cores (more than 1 : 4.75) is disclosed; in EP 1,108,425 the drug loading of less than 14 % of omeprazole in final pharmaceutical formulation and the ratio between omeprazole and inert cores of more than 1:3.6 is disclosed; in WO 04/014345 the drug loading of 6.1 % of omeprazole in final pharmaceutical formulation and the ratio between omeprazole and inert cores of 1 : 2.7 is disclosed.

Commercially available omeprazole products usually revealed drug loading of 10 - 15 % of omeprazole in final pellets, which were comprised in the capsules. By taking SEM (Scanning electron microscope) pictures of those products it was also possible to assess the coating thickness of omeprazole layer in final pellets. The thickness of omeprazole layers in final pellets are approximately less than or about 40 $\mu$m.

In the initial phase of the process of fluid bed coating, there occur frequently troubles such as breaking and scraping of the inert spherical cores, the moisture sensitivity of inert cores (also referred to herein as neutral sugar spheres, or cores) causing problems with controlling the agglomeration and sticking, increased solubility and undesirable agglomerates formation of inert cores. All of the above have great influence on the yield in production of pellets.

In EP 0,277,741 the problem of a breaking and scraping of the inert spherical cores was solved by using simultaneous spraying of inert cores with an aqueous binder and with spraying powder containing benzimidazole compound and low substituted hydroxypropylcellulose (HPC).

In EP 1,108,425 the fluid bed equipment with an inner partition device (wurster) and the manufacturing process without using organic solvents in any of the phase of technological process is described as preferred, cheaper and environmental friendly option for producing pellet formulations composed of an inert core coated with a drug containing layer, coated in turn with an intermediate layer and with a final gastroresistant or enteric layer.

There is a strong competition on omeprazole market and therefore there is a high need and thus an object for improved pharmaceutically active ingredient containing pellets, and for an improved method preparing said pellets, and for dosage forms comprising said pellets.

Summary of the invention

The present invention provides the following aspects, subject-matters and preferred embodiments, which respectively taken alone or in combination, contribute to solving the object of the present invention:

1. Pellet comprising, from the inside to the outside,

- a core comprising one or more pharmaceutically acceptable excipients,
- a pharmaceutically active ingredient layer surrounding the core, wherein said pharmaceutically active ingredient layer comprises one or more pharmaceutically active ingredients, preferably selected from the group consisting of omeprazole or esomeprazole, and pharmaceutically acceptable salts thereof; one or more binders; and optionally one or more further excipients,

wherein the thickness of said pharmaceutically active ingredient layer is at least 50 μm,
wherein the weight ratio of pharmaceutically active ingredient(s) : binder(s) in the pharmaceutically active ingredient layer is in the range of from 3:1 to 5:1.

The "pharmaceutically active ingredient layer" is also called "drug-containing layer", "active layer", or "API-containing layer" and means a layer comprising pharmaceutically active ingredient, preferably omeprazole or esomeprazole. Preferably, the core is an inert core.

2. The pellet according to item 1, wherein the core comprises one or more compounds selected from the group consisting of inorganic salts, metal oxides, organic polymers, such as celluloses, starches and sugars, preferably the core is approximately spherical.

3. The pellet according to any of the preceding items, wherein the core is a non-pareil.

4. The pellet according to any of the preceding items, wherein the drug-containing layer is placed directly onto the core.

5. The pellet according to any of the preceding items, wherein the pharmaceutically active ingredient layer comprises at least 80 wt.-%, preferably 85 wt.-%, more preferably at least 90 wt.-% of pharmaceutically active ingredients and binder.

6. The pellet according to any of the preceding items, wherein the weight ratio of pharmaceutically active ingredient:binders is in the range of from 3.5:1 to 5:1, preferably 3.5:1 to 4.5:1, more preferably 4:1 to 4.5:1.

7. The pellet according to any of the preceding items, wherein the content of pharmaceutically active ingredient in the pellets is 20 to 50 wt.-%, preferably 20 to 40 wt.-%, more preferably 25 to 35 wt.-%, even more preferably 25 to 30 wt.-%.

8. The pellet according to any of the preceding items, wherein the weight ratio of pharmaceutically active ingredients:core is in the range of from 1:1.5 to 1:4, preferably is within the range of from 1:1.5 to 1:3, more preferably from 1:1.8 to 1:2.5, and even more preferably from 1:2 to 1:2.5.

9. The pellet according to any of the preceding items, wherein the thickness of the pharmaceutically active ingredient layer is preferably in a range of from 60 μm to 150 μm, more preferably from 70 μm to 140 μm, and even more preferably from 80 μm to 120 μm at one or more positions.

10. The pellet according to any of the preceding items, wherein the binder content in the pharmaceutically active ingredient layer is at least 15 wt.-% and/or wherein the pharmaceutically active ingredient content in the pharmaceutically active ingredient layer is at least 65 wt.-%.

11. The pellet according to any of the preceding items, wherein the thickness of the pharmaceutically active ingredient layer is measured by scanning electron microscopy (SEM).

12. The pellet according to any of the preceding items, wherein the core comprises one or more pharmaceutically acceptable excipients, preferably selected from group consisting of sugar and maize starch.

13. The pellet according to any of the preceding items, wherein the pharmaceutically active ingredient layer comprise one or more further excipients selected form the group consisting of alkaline reacting substances and surfactants.

14. The pellet according to item 13, wherein the alkaline reacting compound is selected from the group consisting of sodium, potassium, calcium, magnesium and aluminum salts of phosphoric acid; carbonic acid; citric acid or other weak inorganic or organic acids; aluminum, calcium and magnesium hydroxides; magnesium oxide or composite substances, such as $Al_2O_3 \cdot 6MgO \cdot CO_2 \cdot 12H_2O$, $(Mg_6Al_2(OH)16CO_3 \cdot 4H_2O)$, $MgO \cdot Al_2O_3 \cdot 2SiO_2 \cdot nH_2O$ and organic pH-buffering substances such as trihydroxymethylaminomethane.

15. The pellet according to item 13 or 14, wherein the alkaline reacting substance is magnesium oxide.

16. The pellet according to any of the preceding items, wherein the core is a neutral sugar sphere comprising 80.0 - 91.5 wt.-% sucrose and 8.5 - 20 wt.-% maize starch.

17. The pellet according to any of the preceding items, wherein the binder is selected from the group consisting of celluloses, such as hydroxypropyl methylcellulose, hydroxypropyl cellulose and carboxymethyl-cellulose sodium, polyvinyl pyrrolidone; sugars; and starches.

18. The pellet according to any of the preceding items, wherein the binder is hydroxypropyl methylcellulose.

19. The pellet according to any of the preceding items, wherein the surfactant is selected from pharmaceutically acceptable non-ionic or ionic surfactants, such as sodium lauryl sulfate and polysorbate, preferably the surfactant is sodium lauryl sulfate.

20. The pellet according to any of the preceding items, comprising a core and pharmaceutically active ingredient layer with the following composition:

(i) 50 - 70 wt.-%, preferably 55 - 65 wt.-%, more preferably 60 - 65 wt.-% core, and,
(ii) 20 - 40 wt.-%, preferably 25 - 35 wt.-%, more preferably 25 - 30 wt.-% pharmaceutically active ingredients, preferably omeprazole or esomeprazole,

5 - 8 wt.-%, preferably 6 - 7 wt.-% binder, preferably hydroxypropyl methylcellulose,
0.5 - 2.0 wt.-%, preferably 0.5 - 1.5 wt.-% alkaline reacting compound, preferably magnesium oxide,
0.5 - 2.0 wt.-%, preferably 0.5 - 1.5 wt.-% surfactant, preferably sodium lauryl sulfate,

all under (ii) included in a pharmaceutically active ingredient layer.

21. The pellet according to any of the preceding items, comprising a core and pharmaceutically active ingredient layer with the following composition:

(i) 50 - 70 wt.-%, preferably 55 - 65 wt.-%, more preferably 60 - 65 wt.-% a neutral sugar sphere, and
(ii) 20 - 40 wt.-%, preferably 25 - 35 wt.-%, more preferably 25 - 30 wt.-% omeprazole or esomeprazole,

5 - 8 wt.-%, preferably 6 - 7 wt.-% hydroxypropyl methylcellulose,
0.5 - 2.0 wt.-%, preferably 0.5 - 1.5 wt.-% magnesium oxide,
0.5 - 2.0 wt.-%, preferably 0.5 - 1.5 wt.-% sodium lauryl sulfate,

all under (ii) included in a pharmaceutically active ingredient layer.

22. The pellet according to any of the preceding items, wherein the pharmaceutically active ingredient layer is obtainable or obtained by spraying a suspension or solution comprising the ingredients of the drug-containing layer onto the core and performing a drying process, preferably the drug-containing layer is prepared in fluidized bed granulator.

23. The pellet according to any of the preceding items, which is obtainable or obtained by a layering process by using a fluidized bed spray granulator.

24. The pellet according to any of the preceding items, further comprising one or more protective layers above the pharmaceutically active ingredient layer and optionally an enteric coating above the one or more protective layers.

25. The drug-loaded pellet according to any of the preceding items, further comprising an enteric coating above the pharmaceutically active ingredient layer.
In a further preferred embodiment, the pellet comprises, preferably consists of, the core, the pharmaceutically active ingredient layer, the protective layer, and the enteric coating, with the coatings and layers as respectively disclosed elsewhere herein.

26. The pellet according to item 24 or 25, wherein the protective layer is a water soluble or in water rapidly disintegrating layer, or a polymeric, water soluble, film-coating layer, optionally comprising pH-buffering, alkaline compounds.

27. The final drug-loaded pellet according to items 24-26, comprising a core, a pharmaceutically active ingredient layer, a protective layer, and an enteric layer, with the following composition:

(i) 30 - 50 wt.-%, preferably 35 - 45 wt.-% core,
(ii) 15 - 30 wt.-% , preferably 15 - 25 wt.-%, more preferably 17 - 25 wt.-%, even more preferably 20 - 25 wt.-% pharmaceutically active ingredients, preferably omeprazole or esomeprazole,

3 - 6 wt.-%, preferably 3.5 - 5 wt.-% binder, preferably hydroxypropyl methylcellulose,
0.5 - 2.0 wt.-%, preferably 0.5 - 1.5 wt.-% alkaline reacting compound, preferably magnesium oxide,
0.5 - 2.0 wt.-%, preferably 0.5 - 1.5 wt.-% surfactant, preferably sodium lauryl sulfate,

all under (ii) included in a pharmaceutically active ingredient layer,
(iii) 10 - 20 wt.-%, preferably 10 - 18 wt.-% protective layer, preferably comprising talc and povidone, and
(iv) 10 - 40 wt.-%, preferably 13 - 35 wt.-%, more preferably 15 - 30 wt.-% enteric layer, preferably the enteric layer comprises poly(methacrylic acid-co-ethyl acrylate) (Eudragit®) and triethylcitrate.

28. The pellet according to items 24-27, comprising a core, a drug-containing layer, a protective layer, and an enteric layer, with the following composition:

(i) 30 - 50 wt.-%, preferably 35 - 45 wt.-% a neutral sugar sphere core,
(ii) 15 - 30 wt.-% , preferably 15 - 25 wt.-%, more preferably 17 - 25 wt.-%, even more preferably 20 - 25 wt.-% omeprazole or esomeprazole,

3 - 6 wt.-%, preferably 3.5 - 5 wt.-% hydroxypropyl methylcellulose,
0.5 - 2.0 wt.-%, preferably 0.5 - 1.5 wt.-% magnesium oxide,
0.5 - 2.0 wt.-%, preferably 0.5 - 1.5 wt.-% sodium lauryl sulfate,

all under (ii) included in a pharmaceutically active ingredient layer,
(iii) 10 - 20 wt.-%, preferably 10 - 18 wt.-% protective layer, comprising talc and povidone, and
(iv) 10 - 40 wt.-%, preferably 13 - 35 wt.-%, more preferably 15 - 30 wt.-% enteric layer, the enteric layer comprises poly(methacrylic acid-co-ethyl acrylate) (Eudragit®) and triethylcitrate.

29. Process for preparing a pellet according to any of items 1-28, wherein the pharmaceutically active ingredient layer is applied by a layering process using a fluidized bed spray granulator, wherein a suspension or solution comprising the one or more pharmaceutically active ingredients, the binder and the optional further excipients is sprayed onto the core and dried.

30. Use of the pellet of any of items 1 - 28 for preparing a pharmaceutical composition.

31. Pharmaceutical composition comprising pellets of any of items 1-28 and optionally further pharmaceutically acceptable excipients.

32. Dosage form, preferably a capsule, comprising a drug-loaded pellet of any of items 1 to 28, or a pharmaceutical composition of item 31.
In a preferred embodiment, the capsule is a hard capsule.

33. The dosage form according to item 32, wherein the dose of pharmaceutically active ingredients in the dosage form is in the range of from 10 mg to 40 mg, preferably 10 mg or 20 mg or 40 mg.

34. The dosage form according to item 32 or 33, which is a hard capsule.

35. The capsule according to item 32-34, wherein the size of omeprazole 10 mg strength capsule is preferably about 14,3 mm length and about 5.3 mm width, the size of omeprazole 20 mg strength capsule is preferably about 14,3 mm length and about 5.3 mm width or about 15,9 mm length and about 5.8 mm width, the size of omeprazole 40 mg strength capsule is preferably about 18,00 mm length and about 6,3mm width.

36. The dosage form of any of items 32 to 35 for use in a method of treating a disease selected from the group consisting of gastro esophageal reflux disease, peptic ulcer disease, and Zollinger-Ellison syndrome and for prevention of upper gastrointestinal bleeding in people who are at high risk.

Detailed description of the invention

The present invention is now described in more detail by preferred embodiments and examples, which are however presented for illustrative purpose only and shall not be understood as limiting the scope of the present invention in any way.

Within the context of the present invention, it has now unexpectedly been found that a pellet comprising a core comprising one or more pharmaceutically acceptable excipients, a pharmaceutically active ingredient layer surrounding the core, wherein said pharmaceutically active ingredient layer comprises one or more pharmaceutically active ingredients preferably selected from the group consisting of a racemate or enantiomers of 5-methoxy-2-[[(4-methoxy-3,5-dimethyl-2-pyridinyl)methyl]sulfinyl]-1H-benzimidazole, and pharmaceutically acceptable salts thereof; one or more binders; and optionally one or more further excipients, wherein the pharmaceutically active ingredient layer has a certain, specific thickness and a certain, specific weight ratio of the pharmaceutically active ingredient to binders, and wherein the pellet optionally exhibits a certain, specific relative content of pharmaceutically active ingredient based on the total weight of the pellet, and exhibits improved properties e.g. with regard to dissolution and/or handling. Moreover, the manufacturing process of such pellets, pharmaceutical compositions and/or final dosage forms such as capsules can be improved, for instance with regard to time, costs, and/or batch size.

In particular, the present invention can offer an efficient solution for improved manufacturing of omeprazole-comprising pellets and/or pharmaceutical compositions and/or final dosage forms, without the need for upgrading the manufacturing facility with new manufacturing equipment. In the present invention, it has been surprisingly found that manufacturing efficiency can be significantly improved by carefully selecting the weight ratios of pharmaceutically active ingredient (API) to binders, the thickness of the pharmaceutically active ingredient layer (also referred to herein as drug-containing layer, active layer, active agent layer or API layer), and a minimum content of pharmaceutically active ingredients in the pellet based on the total weight of the pellet.

By using the pellets of the present invention, for instance the amount of needed excipients for producing pharmaceutical compositions (such as dosage forms comprising the pharmaceutically active ingredient of the present invention) can be reduced, the size of production batch (namely the number of final dosage forms such as capsules per production batch) can be increased, and/or the production time can be decreased. As the omeprazole market is increasing in the amount of needed final dosage forms (such as capsules), the increase of dosage form number per batch on same equipment is of great importance for the industry.

Comparably high relative loading of pharmaceutically active ingredient and comparably increased thickness of pharmaceutically active ingredient layer, without the pellets being too sticky for proper manufacturing at the same time, was achieved by selection of appropriate ratio between binder and pharmaceutically active ingredient.

The high loading of pharmaceutically active ingredient relative to the pellet weight results in smaller final dosage forms, preferably capsule, comprising pharmacologically efficient amounts of high relative loading of pharmaceutically active ingredients. What would increase a patient compliance.

Further, to overcome the dissolution limitation due to the increased thickness of the pharmaceutically active ingredient layer it has surprisingly been found that increasing binder content until a certain weight ratio of pharmaceutically active ingredient:binder is reached, can enhance the dissolution rate from pharmaceutical composition comprising a core covered by a pharmaceutically active ingredient layer, despite the general expectation that increasing binder amount would slow down the dissolution.

The preferred pharmaceutically active ingredients used in the present invention are omeprazole and esomeprazole, which are drugs used in the treatment of e.g. peptic ulcer disease, and Zollinger-Ellison syndrome. They are also used to prevent upper gastrointestinal bleeding in people who are at high risk.

Omeprazole and esomeprazole are highly sensitive to acid environment of stomach. For this reason medication, final dosage forms or pharmaceutical compositions comprising omeprazole or esomeprazole needs to be protected against stomach acid by means of coating the pharmaceutically active ingredient with enteric polymer. Enteric polymers do not dissolve at low pH and by this protecting the pharmaceutically active ingredient. After the pH increases in small intestine, the enteric polymer dissolves and releases the pharmaceutically active ingredient. For safety and efficacy of patients it is desired and thus aimed at that essentially no release of omeprazole or esomeprazole is achieved in acid environment in order to prevent degradation. At higher pH, a rapid and fast release of omeprazole or esomeprazole is required in order to ensure fast absorption of omeprazole or esomeprazole and consequently pharmacological effect. It is preferred that such enteric formulation is in the form of a multi-unit dosage system, due to a reduced risk of dose dumping and facilitated stomach passage due to a smaller size of the dosage form. The most common dosage form is capsule which is filled with pellets. One of the possibilities to produce such pellets is by applying layers of pharmaceutically active ingredient and excipients onto a neutral sugar spheres or cores.

However, for instance when aiming to increase batch size, the amount of cores could be increased. Such an increased quantity of cores can cause several disadvantages during the manufacturing process, e.g. i) lower drug loading and consequently decreased batch size, longer production time and increased amount of needed excipients; ii) loss of yield due to the friability of cores and therefore increased expense and difficulty of manufacture.

The solution to one or more, preferably all, of the above problems is the reduction of the amount of pellet cores on which omeprazole or esomeprazole is positioned, preferably layered, and increasing binder amount in the pharmaceutically active ingredient layer. By reducing the amount of pellet cores the dissolution profile was reduced and it was surprisingly found that the problem can be solved by providing pellets according to the present invention, namely pellets that exhibit a certain minimum thickness of pharmaceutically active ingredient layer and that comprises a specific, weight ratio of pharmaceutically active ingredients:binders.

The pellets according to the present invention can provide higher pharmaceutically active ingredient loading; reduced amount of needed excipients, increased batch size, reduced production time, smaller size of final dosage form, preferably capsule, improved manufacture efficacy, decreased energy consumption during the manufacturing process, and/or positive impact on environment.

Omeprazole products that are at present commercially available usually revealed a normal drug loading of 10 - 15% of omeprazole in the final pellets, corresponding to a thickness of the pharmaceutically active ingredient layer of up to approximately 40 $\mu$m.

By selecting appropriate excipients and layering technology it has surprisingly been found that there is a possibility of providing pellets that comprise comparably higher pharmaceutically active ingredient loading, and that it is additionally possible to increase the thickness of the pharmaceutically active ingredient layer, e.g. to approximately 100 $\mu$m or even up to approximately 150 $\mu$m. This was achieved by reducing the amount of pellet cores onto which the active agent is positioned, preferably layered.

Additionally, in usual active ingredient layering technologies, a higher ratio of pharmaceutically active ingredient:core is used (for example pharmaceutically active ingredient:neutral core = 1:5 or more). By applying the present invention, this ratio can be reduced even to e.g. 1:2. In other words, by applying the teaching of the present invention, the amount of the pharmaceutically active ingredient per pellet can be increased compared to prior art pellets, the thickness of the pharmaceutically active ingredient layer is increased compared to prior art pellets, and at the same time the stickiness of the pellets can be prevented.

As disclosed elsewhere herein, an important role in achieving such high pharmaceutically active ingredient loading is the selection of an appropriate binder amount, or weight ratio of pharmaceutically active ingredient:binder, respectively. The binder role is to stick particles of pharmaceutically active ingredient on pellet cores during manufacturing, e.g. during layering process. In the present invention it has been shown that increasing binder:omeprazole ratio to a ratio of at least 2:10 is sufficient to achieve appropriate binding of omeprazole onto pellet cores.

| Binder:omeprazole ratio in active layer | Binding of omeprazole onto pellet cores [%] |
|---|---|
| **1:10** | 92 |
| **2:10** | 100 |
| **3:10** | 102 |

Table 1: Impact of the weight ratio of binder:omeprazole on binding of omeprazole onto pellet cores.

Even though the ratio of binder:omeprazole being 2:10 is sufficient for the binding of the active ingredient onto the pellet core during manufacturing process, in the context of the present invention surprising benefits were obtained additionally by increasing the binder concentration.

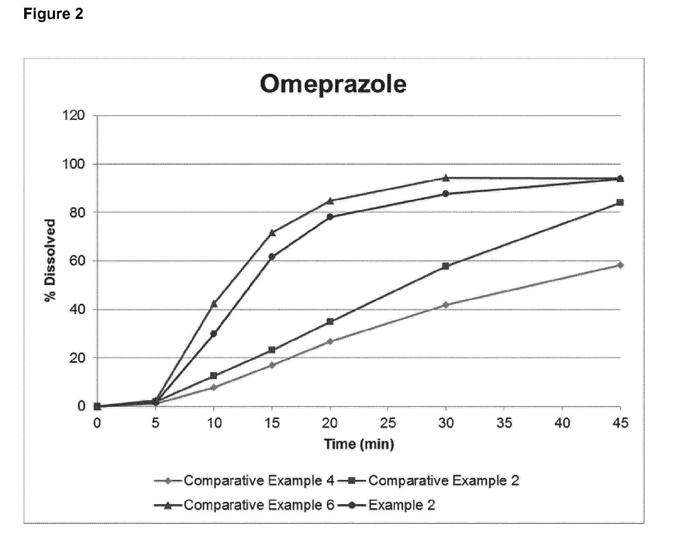

As the thickness of pharmaceutically active ingredient layer (also referred to herein as drug loading) is increased due to the lower amount of neutral pellet cores, the dissolution rate of pharmaceutically active ingredient is decreased, as can be seen when comparing the dissolution rate of Comparative example 4 with the dissolution rate of Comparative example 2 (Fig. 2). This can be contributed to the increased thickness of the pharmaceutically active ingredient layer for comparative example 4 (Fig. 2) which is dissolution limiting factor.

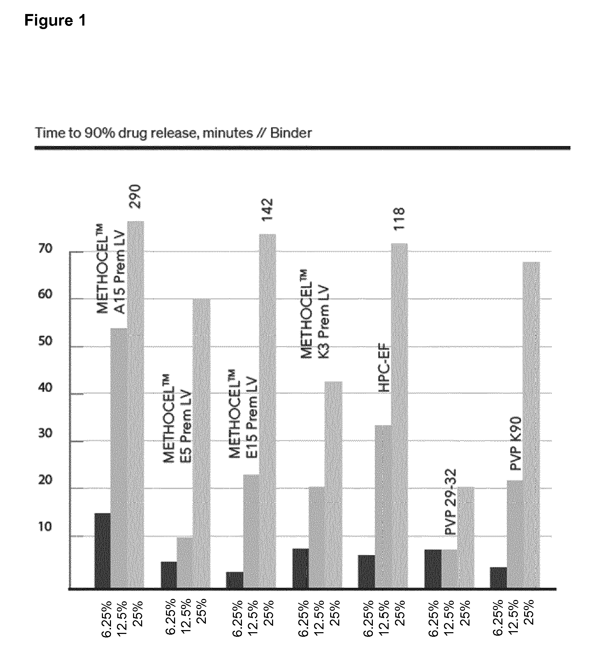

We surprisingly found that increasing the amount of binder enhances dissolution rate from enteric coated pellets. If the amount of binder was high, the dissolution rate was increased, as can be seen when comparing the dissolution rate of Example 2 with the dissolution rate of Comparative example 4 (Fig. 2). As it is generally accepted that increasing binder amount would slow down the dissolution (Fig. 1), this was a surprising effect of the binder.

At the same time, however, it has surprisingly been found in the context of the present invention that the advantageous effects of the present invention can only be achieved if a certain, defined range of weight ratio of pharmaceutically active ingredient :binder is fulfilled. If the content of binder, relatively to the pharmaceutically active ingredient API content, is too high, then disadvantageous properties of the pellet occur. An example of such a disadvantageous property is increased stickiness of the pellets, which can result in severe problems during manufacturing. If the content of binder is too low, then there can occur difficulties with loading the desired high amount of pharmaceutically active ingredient onto a core.

Hence, within the context of the present invention it has now unexpectedly been found that when applying a certain high amount of pharmaceutically active ingredient, and the thickness of the pharmaceutically active ingredient layer being at least 50 $\mu$m at one or more positions, then a certain defined range of weight ratio of pharmaceutically active ingredient layer:binders has to be fulfilled, in order to arrive at pellets that exhibit improved properties.

The present invention thus relates to a pellet comprising a core comprising one or more pharmaceutically acceptable excipients, wherein a pharmaceutically active ingredient layer surrounds the core, wherein said pharmaceutically active ingredient layer comprises one or more pharmaceutically active ingredients selected from the group consisting of enantiomers of 5-methoxy-2-[[(4-methoxy-3,5-dimethyl-2-pyridinyl)methyl]sulfinyl]-1H-benzimidazole, and pharmaceutically acceptable salts thereof; one or more binders; and optionally one or more further excipients. Additionally, the thickness of said pharmaceutically active ingredient layer is at least 50 $\mu$m at one or more positions, the weight ratio of pharmaceutically active ingredient:binders is in the range of from 3:1 to 5:1.

In a preferred embodiment, the weight ratio of pharmaceutically active ingredient:binders is in the range of from 3:1 to 5:1, preferably 3.5:1 to 5:1, more preferably 3.5:1 to 4.5:1, even more preferably 4:1 to 4.5:1.

The one or more pharmaceutically active ingredients(API) are selected from the group consisting of (S)5-methoxy-2-[[(4-methoxy-3,5-di-methyl-2-pyridinyl)methyl]sulphinyl]-1H-benzimidazole, (R)5-methoxy-2-[[(4-methoxy-3,5-di-methyl-2-pyridinyl)methyl]sulphinyl]-1H-benzimidazole (also known as esomeprazole), and pharmaceutically acceptable salts of the aforementioned compounds.

In a further, preferred embodiment, the pharmaceutically active ingredients consist of a mixture of (S)-form and (R)-form, i.e. the active agent is omeprazole. If the pharmaceutically active ingredients consist of a mixture of (S)- and (R)-form, then it is preferred that 40-60% of the pharmaceutically active ingredients are of (S)-form.

Within the context of the present invention it has been found that pellets can be provided that exhibit a comparably high drug loading, i.e. the content of pharmaceutically active ingredients in the pellet is 20 to 50 wt.-%, preferably 20 to 40 wt.-%, more preferably 25 to 35 wt.-%, even more preferably 25 to 30 wt.-%.

In a preferred embodiment, the weight ratio of pharmaceutically active ingredients:core is in the range of from 1:1.5 to 1:4, preferably is within the range of from 1:1.5 to 1:3, more preferably from 1:1.8 to 1:2.5, and even more preferably from 1:2 to 1:2.5.

A high pharmaceutically active ingredient content in pellets of the present invention is in line with an increased thickness of the pharmaceutically active ingredient layer of at least 50 $\mu$m at one or more positions. In preferred embodiments, the thickness of the pharmaceutically active ingredient layer is in a range of from 60 $\mu$m to 150 $\mu$m, more preferably from 70 $\mu$m to 140 $\mu$m, and even more preferably from 80 $\mu$m to 120 $\mu$m at one or more positions. The thickness of any layer that is present in the pellet according to the present invention can be determined by any suitable means that is known to a person skilled in the art. An example of such a suitable means is scanning electron microscopy (SEM).

Within the meaning of the present invention, the term "core" defines a neutral (inert) starter material for pellet preparation and in general encompasses spheres, seeds, pellets, spheroids, granules, beads, particles, and the like. The core that is used for being coated with the pharmaceutically active ingredient layer can be any suitable core that is known to a person skilled in the art, having any suitable size. In a preferred embodiment, the core comprises one or more pharmaceutically acceptable excipients that are selected from the group consisting of inorganic salts, metal oxides, organic polymers, such as celluloses, starches and sugars. In a preferred embodiment, the core is a neutral sugar core. In a further preferred embodiment, the core comprises sugar and/or maize starch.

In a further preferred embodiment, the core is a neutral sugar sphere comprised of 80.0 - 91.5 wt.-% sucrose and 8.5 - 20 wt.-% maize starch.

In an embodiment according to the present invention, the core can have any suitable size. It is however preferred that the core has a mean particle size of from about 250 $\mu$m to about 1000 $\mu$m, preferably from about 400 to about 900 $\mu$m, more preferably from about 600 to about 850 $\mu$m, even more preferably from about 700 to about 800 $\mu$m as determined by sieve analysis]. Additionally, it is preferred that the core has approximately spherical shape. The shape of the core can be assessed by any suitable method that is known to a person skilled in the art, preferably the shape is assessed by applying SEM. Within the meaning of the present invention, the term "approximately spherical shape" denotes that the core has a certain diameter, with a deviation of +/- 20 %.

If the core is not in a sufficiently spherical shape, then suitable measures have to be taken or making the cores sufficiently spherical. Such measures are known to a person skilled in the art and comprise for instance the use of analysis marumerizer.

An approximately spherical shape allows for a more uniform coating of the core with the subsequent layers such as the pharmaceutically active ingredient layer, therefore providing pellets that are improved e.g. with regard to content uniformity, or overall handling.

It is further preferred that the core is inert.

On the core, a pharmaceutically active ingredient layer that surrounds the core is present. It is possible that there are one or more additional layers between the core and the pharmaceutically active ingredient layer. The one or more additional layers that can be present between the core and the pharmaceutically active ingredient layer as disclosed herein can for instance be layer that comprise additional pharmaceutically active ingredient, such as acid-labile pharmaceutically active ingredient, that is not selected from the group consisting of enantiomers of 5-methoxy-2-[[(4-methoxy-3,5-di-methyl-2-pyridinyl)methyl]sulphinyl]-1H-benzimidazole, and pharmaceutically acceptable salts thereof. It is further possible that the layers are coatings that protect the core from mechanical pressure during manufacturing process. It is however preferred that the pharmaceutically active ingredient layer is placed directly on the core, i.e. that there is no further layer between the core and said pharmaceutically active ingredient layer.

The pharmaceutically active ingredient layer comprises, beside the one or more pharmaceutically active ingredients disclosed elsewhere herein, one or more binders, and optionally one or more further excipients.

Binders act as an adhesive to bind together the ingredients of e.g. pharmaceutical compositions to result in the necessary mechanical strength. In general, a person skilled in the art knows which binders are suitable for a certain application or pharmaceutical composition. Preferred binders that are present in the drug-containing layer of the present invention are selected from the group consisting of celluloses, such as hydroxypropyl methylcellulose (HPMC; Hypromellose), hydroxypropyl cellulose (HPC) and carboxymethylcellulose sodium, polyvinyl pyrrolidone (PVP); sugars; and starches. In a preferred embodiment, the binder is hydroxypropyl methylcellulose (HPMC).

The content of pharmaceutically active ingredients in the pellet according to the present invention is at least 15 wt.-%, preferably at least 20 wt.-% t based on the total weight of the pellet. Thus, in a preferred embodiment, the pharmaceutically active ingredient layer comprises at least 80 wt.-%, preferably 85 wt.-%, more preferably at least 90 wt.-% of pharmaceutically active ingredients and binder together. It is additionally preferred that the binder content in the drug-containing layer is at least 15 wt.-%, and/or that the pharmaceutically active ingredient content in the drug-containing layer is at least 65 wt.-%.

Optionally, one or more further excipients, in addition to the pharmaceutically active ingredients and binder, can be present in the drug-containing layer.

The pharmaceutically active ingredient layer optionally comprises one or more further excipients selected from the group consisting of an alkaline reacting compound and one or more surfactants. An alkaline reacting compound is a compound that is able to neutralize acidic compounds. Acidic compounds directly or indirectly interact with pharmaceutically active ingredient that is acid-labile, such as omeprazole, and can thus for instance lead to a degradation of acid-labile compounds. Accordingly, the presence of an alkaline reacting compound is advantageous if there is the possibility that an acid-labile pharmaceutically active ingredient comes into contact with acidic compounds or with an acidic environment, as the alkalizing agent stabilizes the pharmaceutically active ingredient comprising.

In a preferred embodiment, the alkaline reacting compound is selected from the group consisting of sodium, potassium, calcium, magnesium and aluminum salts of phosphoric acid; carbonic acid; citric acid or other weak inorganic or organic acids; aluminum, calcium and magnesium hydroxides; magnesium oxide or composite substances, such as $Al_2O_3 \cdot 6MgO \cdot CO_2 \cdot 12H_2O$, $(Mg_6Al_2(OH)16CO_3 \cdot 4H_2O)$, $MgO \cdot Al_2O_3 \cdot 2SiO_2 \cdot nH_2O$ and organic pH-buffering substances such as trihydroxymethylaminomethane. Preferably, the alkaline reacting substance is magnesium oxide.

In addition to the binder, the pharmaceutically active ingredient layer can additionally comprise one or more surfactants. Surfactants are also called surface-active agents or solubilizing agents, used to disperse pharmaceutically active ingredient in a particular solvent and also enhance wetting properties of the pharmaceutically active ingredient thereby helps either to lower the surface tension of a liquid, allowing easier spreading, and lower the interfacial tension between two liquids or to solubilize the pharmaceutically active ingredient either in composition or *in-situ* at the site of absorption or action. They contain both hydrophobic groups and hydrophilic groups, thus being soluble in both organic solvents and water.

In a preferred embodiment, the surfactant is selected from pharmaceutically acceptable non-ionic or ionic surfactants, such as sodium lauryl sulfate or polysorbate. In a preferred embodiment, the surfactant is sodium lauryl sulfate.

It is possible that the pellet of the present invention does not comprise an enteric layer and/or a protective layer. In one embodiment, the pellet does neither comprise an enteric layer nor a protective layer. In this case, the pellet only consists of a core and a pharmaceutically active ingredient layer as disclosed herein. Such a pellet represents an intermediate product. In a preferred embodiment, the intermediate pellet consists of a core, and a drug-containing layer, with the following composition:

> (i) 50 - 70 wt.-%, preferably 55 - 65 wt.-%, more preferably 60 - 65 wt.-% core, and,
> (ii) 20 - 40 wt.-%, preferably 25 - 35 wt.-percent, more preferably 25 - 30 wt.-% pharmaceutically active ingredients, preferably omeprazole or esomeprazole,
>
>> 5 - 8 wt.-%, preferably 6 - 7 wt.-% binder, preferably hydroxypropyl methylcellulose,
>> 0.5 - 2.0 wt.-%, preferably 0.5 - 1.5 wt.-% alkaline reacting compound, preferably magnesium oxide,

0.5 - 2.0 wt.-%, preferably 0.5 - 1.5 wt.-% surfactant, preferably sodium lauryl sulfate,

all under (ii) included in a pharmaceutically active ingredient layer.

In an even more preferred embodiment, the pellet comprises a core and a pharmaceutically active ingredient layer with the following composition:

(i) 50 - 70 wt.-%, preferably 55 - 65 wt.-%, more preferably 60 - 65 wt.-% a neutral sugar sphere, and
(ii) 20 - 40 wt.-%, preferably 25 - 35 wt.-%, more preferably 25 - 30 wt.-% omeprazole or esomeprazole,

5 - 8 wt.-%, preferably 6 - 7 wt.-% hydroxypropyl methylcellulose,
0.5 - 2.0 wt.-%, preferably 0.5 - 1.5 wt.-% magnesium oxide,
0.5 - 2.0 wt.-%, preferably 0.5 - 1.5 wt.-% sodium lauryl sulfate,
all under (ii) included in a pharmaceutically active ingredient layer.

The pharmaceutically active ingredient layer of the pellet of the present invention can be prepared according to any suitable method or process that is known to a person skilled in the art. In a preferred embodiment, the pharmaceutically active ingredient layer is applied onto the preceding layer or the core, respectively, by a layering process using a fluidized bed spray granulator (e.g. Huettlin HKC 200), wherein a suspension or solution comprising the one or more pharmaceutically active ingredient comprising, the binder and the optional further ingredients is sprayed onto the core and dried. It is preferred that the pharmaceutically active ingredient layer is applied by using a fluidized bed granulator.
For instance, the one or more pharmaceutically active ingredient comprising (in the respective desired amount, for instance 10mg, 20mg, or 40mg), the binder, and if present the surfactant and if present the alkalizing agent are dispersed in water by using a suitable mixer such as a propeller mixer. The cores (e.g. the neutral sugar cores) are loaded into an appropriate fluid bed apparatus (e.g. Huettlin HKC 200). The cores are heated to an appropriate temperature (e.g. 40 °C) for instance by using hot air, and then they are coated with a prepared dispersion comprising the one or more pharmaceutically active ingredient comprising (e.g. omeprazole). Finally, the coated pellets are sieved in order to remove any fines or agglomerates that may be present.
As already disclosed elsewhere, the pellets of the present invention exhibit a reduced portion of agglomerates.
The present invention also refers to the pellets of the present invention, wherein the pharmaceutically active ingredient layer is obtainable or obtained by spraying a suspension or solution comprising the ingredients of the pharmaceutically active ingredient layer onto the core and performing a drying process. In a preferred embodiment, the pharmaceutically active ingredient layer is prepared in fluidized bed granulator.
The present invention also refers to a pellet as disclosed herein, which is obtainable or obtained by a layering process by using a fluidized bed spray granulator.
It is possible that the pellet comprises one or more protective layer(s) above (e.g. placed upon, preferably directly placed upon) the pharmaceutically active ingredient layer (from the inside to the outside). Optionally, there can be an enteric coating above the one or more protective layers.
The protective layer (or protective coating) is generally applied onto the pharmaceutically active ingredient layer to separate the layer containing the pharmaceutically active ingredient (e.g. omeprazole) from an enteric coating layer (also referred to herein as enteric layer). Enteric layers usually comprise gastro-resistant polymers that have free carboxyl groups with acidic characteristics. If acid-labile pharmaceutically active ingredients such as the pharmaceutically active ingredient of the present invention come into contact with these enteric polymers, this could negatively influence the acid-labile pharmaceutically active ingredient such as causing the degradation of said pharmaceutically active ingredient. For this reason, it is preferred that a protective layer is present in order to separate the pharmaceutically active ingredient layer from the enteric layer.
The protective layer can be of any suitable material that is known to a person skilled in the art that is able to protect the active agent from negative influence (such as degradation) from further layers that may be present in the pellet, in particular from enteric layer(s). In a preferred embodiment, the protective layer is a water soluble or in water rapidly disintegrating layer, or a polymeric, water soluble, film-coating layer, optionally containing pH-buffering, alkaline compounds. In an even further preferred embodiment, the protective layer comprises povidone (polyvinylpyrrolidone) and talc.
Enteric coatings are known in the art and are typically designed to not/not substantially dissolve in the stomach. An example of commercially available enteric coating polymer is known under the tradename EUDRAGIT® (Evonik, Germany). For instance, EUDRAGIT®S 100 dissolves at a pH value above 7.0 which corresponds to the conditions as present in the colon. This leads to the effect that the enteric coating does not dissolve in the stomach, but in the colon. A further example of an enteric coating polymer is EUDRAGIT® L100-55 or L30D-55, which dissolve at a pH value above 5.5, which means that it dissolves in the duodenum. Further enteric coating examples and further enteric coating polymers exist.

Preferably the enteric polymer/enteric coating fulfils the requirements for enteric coatings as defined in the European Pharmacopoeia, chapter 2.9.3. (7th Edition 2011 (7.2)).

In one embodiment, the enteric polymer is an anionic polymer or copolymer, preferably at least one selected from the group consisting of enteric cellulose derivatives, enteric acrylic copolymers, enteric maleic copolymers, enteric polyvinyl derivatives and shellac. Even further preferred, the enteric polymer is an anionic polymer with methacrylic acid as a functional group. Further preferred the enteric polymer is an anionic copolymer selected from the group consisting of methacrylic acid/ethylacrylate, methacrylic acid/methyl methacrylate, and methacrylic acid/methylacrylate/methyl methacrylate.

In one embodiment, the anionic polymer or mixture of anionic polymers is used in combination with an methacrylate copolymer having neutral ester groups (e.g. $R=COOCH_3$ or $R=COOC_4H_9$) or trimethylammonioethyl groups ($R=COOCH_2CH_2N^+(CH_3)_3Cl$). Methacrylate copolymers having a neutral ester are insoluble and pH-independent and can be used to provide a time controlled release dosage form, wherein e.g. after dissolution of the anionic polymer a matrix of the insoluble polymer remains and the medicinal substance can be washed out of this matrix.

In a preferred embodiment, the enteric polymer/enteric coating layers dissolve(s) above a pH of 5.5. A dosage form comprising such enteric coating layers will therefore release the pharmaceutically active ingredient directly after leaving the stomach, e.g. in the duodenum. In another embodiment of the invention, the enteric polymer/enteric coating layers dissolve(s) at a pH of between 5.5 and 7.0.

In another embodiment, the enteric polymer/coating layers dissolve(s) at a pH above 7.0. In another embodiment, the enteric polymer/enteric coating layers dissolve(s) at a pH of between 5.5 and 6.5, most preferred at a pH of between 5.5 and 6.0.

Preferred enteric cellulose derivatives according to the present invention are hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose or ethylhydroxyethylcellulose phthalate.

Suitable hydroxymethylethylcellulose phthalates are also sold under the tradename HP50® and HP55® (Shin-Etsu, Japan).

Preferred enteric acrylic copolymers are styrene/acrylic acid copolymer, methyl acrylate/ acrylic acid copolymer, methyl acrylate/ methacrylic acid copolymer, butyl acrylate/ styrene / acrylic acid copolymer, methacrylic acid/ methyl methacrylate copolymer, methacrylic acid/ ethylacrylate copolymer or methyl acrylate/ methacrylic acid/ octyl acrylate copolymer, or methacrylic acid/methylacrylate/methyl methacrylate.

Suitable anionic polymers or copolymers with methacrylic acid as a functional group are sold under the tradename EUDRAGIT® (Evonik, Germany): EUDRAGIT® L100-55 (sold in powder form; dissolution above pH 5.5), EUDRAGIT® L30 D-55 (sold as aqueous dispersion, 30%; dissolution above pH 5.5), EUDRAGIT® L100 (sold in powder form; dissolution above pH 6.0) or EUDRAGIT® L12,5 (sold as organic solution, 12.5%; dissolution above pH 6.0), EUDRAGIT® S100 (sold in powder form; dissolution above pH 7.0), EUDRAGIT® S12,5 (sold as organic solution, 12.5%; dissolution above pH 7.0) or EUDRAGIT® FS 30D (sold as aqueous dispersion, 30%; dissolution above pH 7.0).

Suitable methacrylate copolymers having neutral ester groups are also sold under the tradename EUDRAGIT® (Evonik, Germany): EUDRAGIT® NE 30D/ 40D /NM 30D and methacrylate copolymers having trimethylammonioethyl groups are sold under the tradename EUDRAGIT® (Evonik, Germany): EUDRAGIT® RL100, EUDRAGIT® RL PO/ RS PO, EUDRAGIT® RL 30D/RS 30D, and EUDRAGIT® RL12,5/ RS12,5.

Preferred enteric maleic copolymers are vinylacetate/ maleic acid anhydride copolymer, styrene/ maleic acid anhydride copolymer, styrene/ maleic acid monoester copolymer, vinylmethylether/ maleic acid anhydride copolymer, ethylene/ maleic acid anhydride copolymer, vinylbutylether/ maleic acid anhydride copolymer, acrylonitrile/ methyl acrylate/ maleic acid anhydride copolymer or butyl acrylate/ styrene/ maleic acid anhydride copolymer.

Preferred enteric polyvinyl derivatives are polyvinyl alcohol phthalate, polyvinylacetal phthalate, polyvinyl butylate phthalate or polyvinylacetoacetal phthalate

One possible coating technique for applying a coating composition, notably a drug-containing layer composition, a protective coating composition, or an enteric coating composition, onto a surface of a particle such as e.g. a core, a granule, a dosage formulation or the like is the technique of spray coating. When applying the process of spray coating, the coating composition can e.g. be sprayed onto fluidized particles, e.g. the drug-loaded pellets.

In a preferred embodiment, the respective layers are applied by spray coating.

General principles for applying enteric coatings are known to a person skilled in the art and are for instance described in the Eudragit® Application Guidelines (10th Revised Edition, 06/2008) or elsewhere.

In a further embodiment, the pellet comprises an enteric coating above the one or more protective layers. Thus, it is a further embodiment that the pellet also comprises a protective layer and an enteric layer, in addition to the core and the pharmaceutically active ingredient layer. Such a pellet is also referred to herein as a so-called final pellet (also referred to herein as enteric coated pellet).

In a preferred embodiment, the final pellet comprises a core, a pharmaceutically active ingredient layer, a protective layer, and an enteric layer, with the following composition:

(i) 30 - 50 wt.-%, preferably 35 - 45 wt.-% core,
(ii) 15 - 30 wt.-% , preferably 15 - 25 wt.-%, more preferably 17 - 25 wt.-%, even more preferably 20 - 25 wt.-% pharmaceutically active ingredients, preferably omeprazole or esomeprazole,

3 - 6 wt.-%, preferably 3.5 - 5 wt.-% binder, preferably hydroxypropyl methylcellulose,
0.5 - 2.0 wt.-%, preferably 0.5 - 1.5 wt.-% alkaline reacting compound, preferably magnesium oxide,
0.5 - 2.0 wt.-%, preferably 0.5 - 1.5 wt.-% surfactant, preferably sodium lauryl sulfate,

all under (ii) included in a pharmaceutically active ingredient layer,
(iii) 10 - 20 wt.-%, preferably 10 - 18 wt.-% protective layer, preferably comprising talc and povidone, and
(iv) 10 - 40 wt.-%, preferably 13 - 35 wt.-%, more preferably 15 - 30 wt.-% enteric layer, preferably the enteric layer comprises poly(methacrylic acid-co-ethyl acrylate) (Eudragit®) and triethylcitrate.

In a preferred embodiment, the final pellet comprises a core, a pharmaceutically active ingredient layer a protective layer, and an enteric layer, and with the following composition:

(i) 30 - 50 wt.-%, preferably 35 - 45 wt.-% a neutral sugar sphere core,
(ii) 15 - 30 wt.-% , preferably 15 - 25 wt.-%, more preferably 17 - 25 wt.-%, even more preferably 20 - 25 wt.-% omeprazole or esomeprazole,

3 - 6 wt.-%, preferably 3.5 - 5 wt.-% hydroxypropyl methylcellulose,
0.5 - 2.0 wt.-%, preferably 0.5 - 1.5 wt.-% magnesium oxide,
0.5 - 2.0 wt.-%, preferably 0.5 - 1.5 wt.-% sodium lauryl sulfate,

all under (ii) included in a pharmaceutically active ingredient layer,
(iii) 10 - 20 wt.-%, preferably 10 - 18 wt.-% protective layer, comprising talc and povidone, and
(iv) 10 - 40 wt.-%, preferably 13 - 35 wt.-%, more preferably 15 - 30 wt.-% enteric layer, the enteric layer comprises poly(methacrylic acid-co-ethyl acrylate) (Eudragit®) and triethylcitrate.

The present invention also refers to the use of the pellets as disclosed herein for preparing a pharmaceutical composition. This pharmaceutical composition can additionally comprise one or more further pharmaceutically acceptable excipients. These pharmaceutically acceptable excipients are preferably selected from the group consisting of diluents, binders, fillers, disintegrants, lubricants, controlled release (CR) agents, sweeteners, glidants, flavourings and colouring agents, wherein:

the fillers are selected from the group consisting of different grades of starches, such as maize starch, potato starch, rice starch, wheat starch, pregelatinized starch, fully pregelatinized starch; cellulose, such as microcrystalline cellulose or silicified microcrystalline cellulose; mannitol, erythritol; lactose, such as lactose monohydrate, lactose anhydrous, spray dried lactose or milled lactose; calcium salts, such as calcium hydrogenphosphate; sorbitol, and xylitol; particularly preferably the fillers are selected from the group consisting of pregelatinized starch, microcrystalline cellulose, silicified microcrystalline cellulose, lactose monohydrate, spray dried lactose, and milled lactose; the disintegrants are selected from the group consisting of carmellose calcium, carboxymethylstarch sodium, croscarmellose sodium (cellulose carboxymethylether sodium salt, crosslinked), starch, and its derivatives such as sodium starch glycolate, crosslinked polyvinylpyrrolidone (crospovidone), and low-substituted hydroxypropylcellulose; particularly preferably the disintegrants are selected from the group consisting of sodium starch glycolate, croscarmellose sodium and crospovidone; the lubricants are selected from the group consisting of stearic acid, talc, glyceryl behenate, sodium stearyl fumarate and magnesium stearate; particularly preferably the lubricant are magnesium stearate and sodium stearyl fumarate; the binders are selected from the group consisting of polyvinyl pyrrolidone (Povidone), copolymers of vinylpyrrolidone with other vinyl derivatives (Copovidone), hydroxypropyl methylcellulose, methylcellulose, hydroxypropylcellulose, powdered acacia, gelatin, guar gum, carbomer such as carbopol, polymethacrylates and pregelatinized starch; the diluents are selected from carbohydrates such as monosaccharides like glucose, oligosaccharides like sucrose, anhydrous lactose, lactose monohydrate or milled lactose, and sugar alcohols like sorbitol, mannitol, erythrol, and xylitol; particularly preferably the diluent is selected from the group consisting of sorbitol and milled lactose; the glidants are selected from the group consisting of colloidal silica, hydrophobic colloidal silica and magnesium trisilicate, such as talc; particularly preferably the glidants are selected from the group consisting of colloidal silica and hydrophobic colloidal silica;

the controlled release (CR) agents for the production of matrix based CR solid dosage forms are preferably selected from the group consisting of high viscosity water soluble polymers such as hydroxypropyl cellulose and hypromellose, and lypophilic matrix forming agents such as glyceryl behenate; and/or

the sweeteners are selected from the group consisting of aspartame, saccharin sodium, dipotassium glycyrrhizinate, aspartame, stevia, thaumatin, and the like; preferably the excipients are microcrystalline cellulose, silicified microcrystalline cellulose, milled lactose, spray dried lactose, croscarmellose sodium, sodium starch glycolate, low substituted hydroxypropylcellulose, crospovidone, magnesium stearate, and sodium stearyl fumarate.

The present invention further refers to a dosage from that comprises a pellet as described herein. Preferably, the dosage from represents a solid oral dosage form, preferably the dosage form is a tablet or a capsule. In a preferred embodiment, the dosage from is a hard capsule.

In a preferred embodiment, the dosage form of the present invention comprises a dose of pharmaceutically active ingredients in the range of from 10 mg to 40 mg, e.g. 10 mg, 20 mg or 40 mg, preferably the dose of pharmaceutically active ingredients in the dosage form is 40 mg.

In a preferred embodiment, capsules, preferably hard capsules, comprising omeprazole have the following sizes:

Table 3: Capsule sizes

| Omeprazole strength | Capsule size number | Capsule length | Capsule width |
| --- | --- | --- | --- |
| 10 mg | 4 | 14,3 mm | 5,3 mm |
| 20 mg | 4 or 3 | 14,3 mm or 15,9 mm | 5,3 mm or 5,8 mm |
| 40 mg | 2 | 18,0 mm | 6,3 mm |

Finally, the present invention refers to the dosage form of the present invention for use in a method of treating a disease selected from the group consisting of gastro esophageal reflux disease, peptic ulcer disease, and Zollinger-Ellison syndrome and for prevention of upper gastrointestinal bleeding in people who are at high risk.

Methods

Method for determining particle size:

The following method can be used for determining the mean particle size, e.g. the mean particle size of the core used in the present invention: European Pharmaocopeia, 7th Edition 2011 (7.2), Chapter 2.9.12: "Sieve analysis", or European Pharmaocopeia, 7th Edition 2011 (7.2), Chapter 2.9.31: Particle size analysis by laser light diffraction using Malvern Mastersizer 2000 and a dry dispersion system

Description f the figures

Fig. 1: This figure shows common general knowledge about the impact of binder on drug release. In particular, Fig. 1 depicts the time to 90 % drug release from compressed tablets containing the pharmaceutically active ingredient niacinamide and prepared using roller compaction at 3 tons pressure. Fig. 1 is taken from "Wet and Dry granulation Binder - A Technical Review by Dow".

Fig. 2: This figure shows the dissolution of enteric coated pellets of omeprazole in 900 mL phosphate buffer, pH 6.8 after 2 hrs in 0.1 M HCl; Apparatus 1, 100 rpm (USP).

The applied dissolution method is designed to mimic gastrointestinal environment where the dosage form first reaches the stomach where acidic environment is present and then, after one to two hours, the dosage form passes to duodenum and upper intestinal tract where pH conditional are around pH 7.

First stage is 0.1 M HCl (pH 1) where gastric resistance is challenged and immediate release part of formulation is dissolved. At second stage phosphate buffer, pH 6.8, is applied.

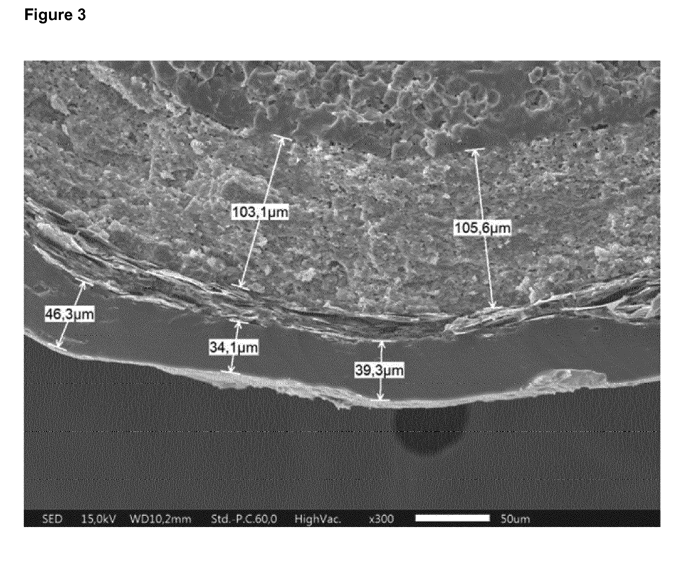

Fig. 3: This figure shows an SEM picture of a relatively high pharmaceutically active ingredient loaded pellet according to the present invention, showing coating thickness of approximately 100 μm for active layer and 40 μm for enteric layer.

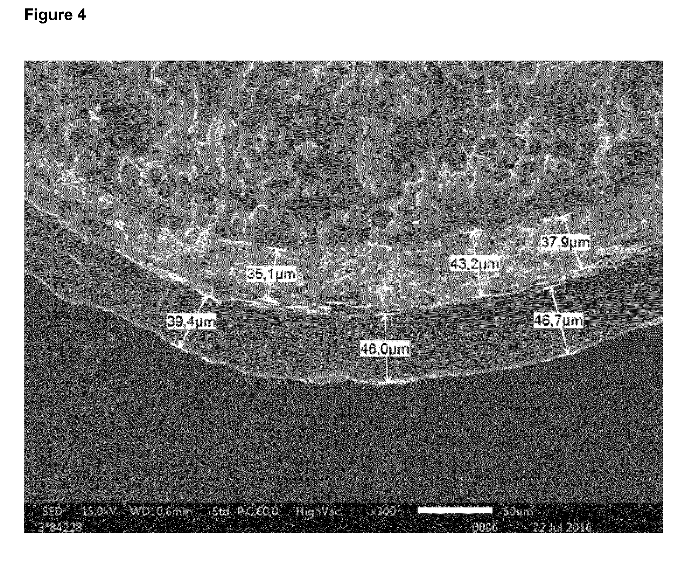

Fig. 4: This figure shows an SEM picture of pellets prepared according to comparative example 2 showing coating thickness of normal loading with approximate omeprazole-layer and enteric coating-layer thickness of 40 μm.

Examples:

In order to illustrate the present invention, pellets comprising an active layer have been prepared. Examples 1, 2, 3 and 4 are according to the present invention, and Comparative Examples 1 to 6 represent comparative examples. The prepared pellets exhibit the following active layer thickness (Table 3) and the following API (omeprazole):neutral core ratios, and API (omeprazole):binder ratios (Table 4):

|  | Omeprazole layer thickness | Enteric layer thickness |
|---|---|---|
| Example 1 and Example 2 | 100 μm | 40 μm |
| Comparative example 1 and 2 | 40 μm | 40 μm |
| Comparative example 3 and 4 | 100 μm | 40 μm |
| Comparative example 5 and 6 | 70 μm | 40 μm |

Table 3: Thickness of omeprazole layer and enteric coating layer as determined by SEM.

|  | Omeprazole:Neutral cores ratio | Omeprazole:Binder ratio |
|---|---|---|
| Example 1, 2, 3 and 4 | 1 : 2.2 | 10 : 2.3 |
| Comparative example 1 and 2 | 1 : 7 | 10 : 1.3 |
| Comparative example 3 and 4 | 1 : 2.2 | 10 : 1.3 |
| Comparative example 5 and 6 | 1 : 3.6 | 10 : 4 |

Table 4: Pharmaceutically active ingredient (omeprazole):neutral core ratios and pharmaceutically active ingredient (omeprazole):binder ratios as used in the examples.

**Example 1:**

Preparation of omeprazole-loaded pellets comprising neutral core and omeprazole layer wherein high omeprazole loading and higher amount of hypromellose was used.

| Substance | Amount per capsule (mg) |
|---|---|
| Omeprazole | 40.0 |
| Neutral sugar cores | 86.7 |
| Hypromellose (HPMC) (binder) | 9.0 |
| *Sodium lauryl sulfate (Surfactant)* | *1.5* |
| *Magnesium oxide (Alkalizing agent)* | *2.0* |
| Total mass (mg) | 139.2 |

Omeprazole/neutral cores ratio = 1:2.2

Omeprazole, Hypromellose (hydroxypropylmethylcellulose; HPMC), Sodium lauryl sulfate and magnesium oxide was dispersed in water using propeller mixer. Neutral sugar cores were loaded into fluid bed apparatus (Huettlin HKC 200). First, neutral pellet cores were heated to 40°C by hot air and then they were coated with prepared omeprazole dispersion. Finally, coated pellets were sieved to remove any fines or agglomerates.
Presented composition can be proportionally adjusted also for 10 and 20 mg doses.

**Example 2:**

Preparation of final pellets that in addition to the omeprazole layer further comprise a protective coating (layer) and an enteric coating (layer)..

| Substance | Amount per capsule (mg) |
|---|---|
| Omeprazole loaded pellet from example 1 | 139.2 |
| Protective layer | 30.0 |
| Enteric layer | 30.8 |
| Total mass (mg) | 200.0 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.
Presented composition can be proportionally adjusted also for 10 and 20 mg doses.
Fig. 2 shows the dissolution profile of enteric coated pellets of Example 2.
Fig. 3 shows an SEM picture of the final pellet prepared as described in Example 2, from which the coating thickness of the omeprazole layer can be easily measured.

**Example 3:**

Preparation of final pellets that in addition to the omeprazole layer further comprise a protective coating (layer) and an enteric coating (layer)..

| Substance | Amount per capsule (mg) |
|---|---|
| Omeprazole loaded pellet from example 1 | 139.2 |
| Protective layer | 30.0 |
| Enteric layer | 35.0 |
| Total mass (mg) | 204.2 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.
Presented composition can be proportionally adjusted also for 10 and 20 mg doses.

**Example 4:**

Preparation of final pellets that in addition to the omeprazole layer further comprise a protective coating (layer) and an enteric coating (layer)..

| Substance | Amount per capsule (mg) |
|---|---|
| Omeprazole loaded pellet from example 1 | 139.2 |

(continued)

| Substance | Amount per capsule (mg) |
|---|---|
| Protective layer | 30.0 |
| Enteric layer | 70.0 |
| Total mass (mg) | 239.2 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.

Presented composition can be proportionally adjusted also for 10 and 20 mg doses.

**Comparative Example 1:**

Preparation of standard omeprazole-loaded pellets comprising neutral core and omeprazole layer, wherein relatively low omeprazole loading and relatively low amount of hypromellose was used.

| Substance | Amount per capsule (mg) |
|---|---|
| Omeprazole | 40.0 |
| Neutral sugar cores | 280.0 |
| Hypromellose (Binder) | 5.0 |
| Sodium lauryl sulfate (Surfactant) | 1.5 |
| Magnesium oxide (Alkalizing agent) | 2.0 |
| Total mass (mg) | 328.5 |

## Omeprazole/Neutral cores ratio = 1:7

Omeprazole, Hypromellose, sodium lauryl sulfate and magnesium oxide were dispersed in water using propeller mixer. Neutral sugar cores were loaded into fluid bed apparatus (Huettlin HKC 5). First, neutral pellet cores were heated to 40°C by hot air and then they were coated with prepared omeprazole dispersion. Finally, coated pellets were sieved to remove any fines or agglomerates.

Presented composition can be proportionally adjusted also for 10 and 20 mg doses.

**Comparative Example 2:**

Preparation of final pellets that in addition to the omeprazole layer further comprise a protective coating (layer) and an enteric coating (layer).

| Substance | Amount per capsule (mg) |
|---|---|
| Omeprazole loaded pellet from comparative example 1 | 328.5 |
| Protective layer | 30.0 |
| Enteric layer | 85 |
| Total mass (mg) | 443.5 |

Protective coating was prepared by mixing talc and Povidone in water and enteric coating was prepared by mixing Eudragit L 30 D 55 and triethylcitrate. Omeprazole loaded pellets of comparative example 1 were coated first with protective coat, followed by enteric coat. Finally coated pellets were sieved to remove any fines or agglomerates.

Presented composition can be proportionally adjusted also for 10 and 20 mg doses.

Fig. 2 shows the dissolution profile of enteric coated pellets of comparative example 2.

Fig. 4 shows an SEM picture of the final pellet prepared as described in comparative example 2.

**Comparative Example 3:**

Preparation of omeprazole-loaded pellets comprising neutral core and omeprazole layer, wherein relatively high omeprazole loading and relatively low amount of hypromellose was used.

| Substance | Amount per capsule (mg) |
| --- | --- |
| Omeprazole | 40.0 |
| Neutral sugar cores | 86.7 |
| Hypromellose (binder) | 5.0 |
| *Sodium lauryl sulfate (Surfactant)* | *1.5* |
| *Magnesium oxide (Alkalizing agent)* | *2.0* |
| Total mass (mg) | 134.2 |

## Omeprazole/Neutral cores ratio = 1:2.2

Omeprazole, Hypromellose, sodium lauryl sulfate and magnesium oxide were dispersed in water using propeller mixer. Neutral sugar cores were loaded into fluid bed apparatus (Huettlin HKC 5). First, neutral pellet cores were heated to 40°C by hot air and then they were coated with the prepared omeprazole dispersion. Finally, the coated pellets were sieved to remove any fines or agglomerates.

Presented composition can be proportionally adjusted also for 10 and 20 mg doses.

**Comparative Example 4:**

Preparation of final pellets that in addition to the omeprazole layer further comprise a protective coating (layer) and an enteric coating (layer).

| Substance | Amount per capsule (mg) |
| --- | --- |
| Omeprazole loaded pellet from comparative example 3 | 134.2 |
| Protective layer | 30.0 |
| Enteric layer | 30.8 |
| Total mass (mg) | 195.0 |

Protective coating was prepared by mixing talc and Povidone (polyvinylpyrrolidone) in water, and enteric coating was prepared by mixing Eudragit L 30 D 55 and triethylcitrate (TEC). Omeprazole loaded pellets of comparative example 3 were coated first with protective coat, followed by coating with the enteric coat. Finally, the coated pellets were sieved to remove any fines or agglomerates.

Presented composition can be proportionally adjusted also for 10 and 20 mg doses.

Fig. 2 shows the dissolution profile of enteric coated pellets of comparative example 4.

**Comparative Example 5:**

Preparation of omeprazole-loaded pellets comprising neutral core and omeprazole layer, wherein medium omeprazole loading and very high amount of hypromellose was used.

| Substance | Amount per capsule (mg) |
| --- | --- |
| Omeprazole | 40.0 |

(continued)

| Substance | Amount per capsule (mg) |
|---|---|
| Neutral sugar cores | 143 |
| Hypromellose | 16.0 |
| *Sodium lauryl sulfate (Surfactant)* | *1.5* |
| *Magnesium oxide (Alkalizing agent)* | *2.0* |
| Total mass (mg) | 202.50 |

## Omeprazole/Neutral cores ratio = 1:3.6

Omeprazole, Hypromellose, sodium lauryl sulfate and magnesium oxide were dispersed in water using propeller mixer. Neutral sugar cores were loaded into fluid bed apparatus (Huettlin HKC 5). First, neutral pellet cores were heated to 40°C by hot air and then they were coated with prepared omeprazole dispersion. Finally, coated pellets were sieved to remove any fines or agglomerates.

Presented composition can be proportionally adjusted also for 10 and 20 mg doses.

**Comparative Example 6:**

Preparation of final pellets that in addition to the omeprazole layer further comprise a protective coating (layer) and an enteric coating (layer).

| Substance | Amount per capsule (mg) |
|---|---|
| Omeprazole loaded pellet from comparative example 5 | 202.5 |
| Protective layer | 30.0 |
| Enteric layer | 52.5 |
| Total mass (mg) | 285.0 |

Protective coating was prepared by mixing talc and Povidone in water and enteric coating was prepared by mixing Eudragit L 30 D 55 and triethylcitrate. Omeprazole loaded pellets of comparative example 5 were coated first with protective coat, followed by enteric coat. Finally coated pellets were sieved to remove any fines or agglomerates.

Presented composition can be proportionally adjusted also for 10 and 20 mg doses.

Fig. 2 shows the dissolution profile of enteric coated pellets of comparative example 6.

**Increase in batch size in number of produced pieces**

As is shown in the following table (Table 5), by applying the present invention the manufacturing procedure can be improved. This is due to increasing the omeprazole loading on pellet cores, thus increasing the omeprazole layer on said cores. This, in turn, is achieved by reducing the amount of excipients and increasing the batch size in number of produced capsules produced on the same equipment. This significantly improves manufacturing effectiveness. As is shown in the table below (Table 5), a significant increase in batch size in terms of produced pieces is possible for the same equipment (batch size in kg).

| | Example 1 and 2 formulation | Comparative example 1 and 2 formulation |
|---|---|---|
| Batch size | 100 kg | 100 kg |
| Batch size (pieces) | 718.391 | 292.826 |

Table 5: Theoretical increase in capsule number at the same batch size weight.

**Binder impact on formation of agglomerates**

The binder has an important role in binding the API on the pellet cores. Thus, it can also bind together two or more pellets which however is not desired as such agglomerates are rejected due to the quality characteristics. Table 6 below shows the portion of agglomerates that is present when using different amounts (ratios) of binder.

|  | Omeprazole:Neutral cores ratio | Omeprazole:Binder ratio | Portion of agglomerates |
|---|---|---|---|
| Example 1 and 2 | 1 : 2.1 | 10 : 2.25 | 0 % |
| Comparative Example 5 and 6 | 1 : 3.6 | 10 : 4 | 25 % |

**Claims**

1. Pellet comprising

   - a core comprising one or more pharmaceutically acceptable excipients,
   - a pharmaceutically active ingredient layer surrounding the core, wherein said pharmaceutically active ingredient layer comprises one or more pharmaceutically active ingredients, preferably selected from the group consisting of omeprazole, esomeprazole, and pharmaceutically acceptable salts thereof; one or more binders; and optionally one or more further excipients,

   wherein the thickness of said pharmaceutically active ingredient layer is more than 50 $\mu$m at one or more positions, and
   wherein the weight ratio of active agent(s) : binder(s) is in the range of from 3:1 to 5:1.

2. The pellet according to claim 1, wherein the core comprises of one or more compounds selected from the group consisting of inorganic salts, metal oxides, organic polymers, such as celluloses, starches and sugars, preferably the core is approximately spherical.

3. The pellet according to claim 1 or 2, wherein the weight ratio of pharmaceutically active ingredients:binders is in the range of from 3.5:1 to 5:1, preferably 3.5:1 to 4.5:1, more preferably 4:1 to 4.5:1.

4. The pellet according to any of the preceding claims, wherein the weight ratio of pharmaceutically active ingredients:core is in the range of from 1:1.5 to 1:4, preferably is within the range of from 1:1.5 to 1:3, more preferably from 1:1.8 to 1:2.5, and even more preferably from 1:2 to 1:2.5.

5. The pellet according to any of the preceding claims, wherein the thickness of the pharmaceutically active ingredient layer is preferably in a range of from 60 $\mu$m to 150 $\mu$m, more preferably from 70 $\mu$m to 140 $\mu$m, and even more preferably from 80 $\mu$m to 120 $\mu$m at one or more positions.

6. The pellet according to any of the preceding claims, wherein the binder content in the drug-containing layer is at least 15 wt.-% and/or wherein the pharmaceutically active ingredient content in the drug-containing layer is at least 65 wt.-%.

7. The pellet according to any of the preceding claims, wherein the pharmaceutically active ingredient layer comprise one or more further excipients selected form the group consisting of alkaline reacting substances and surfactants.

8. The pellet according to any of the preceding claims, wherein the binder is selected from the group consisting of celluloses, such as hydroxypropyl methylcellulose, hydroxypropyl cellulose and carboxymethyl-cellulose sodium, polyvinyl pyrrolidone; sugars; and starches; preferably the binder is hydroxypropylmethylcellulose (HPMC).

9. The pellet according to any of the preceding claims, comprising a core and pharmaceutically active ingredient layer with the following composition:

(i) 50 - 70 wt.-%, preferably 55 - 65 wt.-%, more preferably 60 - 65 wt.-% core, and,
(ii) 20 - 40 wt.-%, preferably 25 - 35 wt.-percent, more preferably 25 - 30 wt.-% pharmaceutically active ingredients, preferably omeprazole or esomeprazole,

>5 - 8 wt.-%, preferably 6 - 7 wt.-% binder, preferably hydroxypropyl methylcellulose,
>0.5 - 2.0 wt.-%, preferably 0.5 - 1.5 wt.-% alkaline reacting compound, preferably magnesium oxide,
>0.5 - 2.0 wt.-%, preferably 0.5 - 1.5 wt.-% surfactant, preferably sodium lauryl sulfate,

all under (ii) included in a pharmaceutically active ingredient layer.

10. The pellet according to any of the preceding claims, comprising a core, a pharmaceutically active ingredient layer, a protective layer, and an enteric layer, with the following composition:

(i) 30 - 50 wt.-%, preferably 35 - 45 wt.-% core,
(ii) 15 - 30 wt.-% , preferably 15 - 25 wt.-%, more preferably 17 - 25 wt.-%, even more preferably 20 - 25 wt.-% pharmaceutically active ingredients, preferably omeprazole or esomeprazole,

>3 - 6 wt.-%, preferably 3.5 - 5 wt.-% binder, preferably hydroxypropyl methylcellulose,
>0.5 - 2.0 wt.-%, preferably 0.5 - 1.5 wt.-% alkaline reacting compound, preferably magnesium oxide,
>0.5 - 2.0 wt.-%, preferably 0.5 - 1.5 wt.-% surfactant, preferably sodium lauryl sulfate,

all under (ii) included in a pharmaceutically active ingredient layer,
(iii) 10 - 20 wt.-%, preferably 10 - 18 wt.-% protective layer, preferably comprising talc and povidone, and
(iv) 10 - 40 wt.-%, preferably 13 - 35 wt.-%, more preferably 15 - 30 wt.-% enteric layer, preferably the enteric layer comprises poly(methacrylic acid-co-ethyl acrylate) (Eudragit®) and triethylcitrate.

11. Process for preparing a pellet according to any of claims 1 to 10, wherein the pharmaceutically active ingredient layer is applied by a layering process using a fluidized bed spray granulator, wherein a suspension or solution comprising the one or more pharmaceutically active ingredients, the binder and the optional further excipients is sprayed onto the core and dried.

12. Use of the pellet of any of claims 1 to 10 for preparing a pharmaceutical composition.

13. Pharmaceutical composition comprising pellets of any of claims 1 to 10, and optionally further pharmaceutically acceptable excipients.

14. Dosage form comprising a pellet of any of claims 1 to 10, or a pharmaceutical composition of claim 13.

15. The dosage form of claim 14 for use in a method of treating a disease selected from the group consisting of gastro esophageal reflux disease, peptic ulcer disease, and Zollinger-Ellison syndrome and for prevention of upper gastrointestinal bleeding in people who are at high risk.

**Figure 1**

Time to 90% drug release, minutes // Binder

**Figure 2**

**Figure 3**

**Figure 4**

Europäisches Patentamt
European Patent Office
Office européen des brevets

# EUROPEAN SEARCH REPORT

Application Number
EP 16 18 7705

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/041276 A1 (JUBILANT ORGANOSYS LTD [IN]; RAJAN GOPAL [IN]; KUMAR PRATIK [IN]; MUKH) 15 April 2010 (2010-04-15) | 1-3,6,8, 11-15 | INV. A61K9/16 A61K31/00 |
| A | * page 2, line 33 - page 4, line 23 * * page 4, lines 18-21 * ----- | 4,5,7,9, 10 | |
| X | EP 0 519 870 A1 (SPIRIG AG [CH]) 23 December 1992 (1992-12-23) | 1-3,6,8, 11-14 | |
| A | * pages 7-8; example 1 * ----- | 4,5,7,9, 10,15 | |

TECHNICAL FIELDS SEARCHED (IPC)

A61K

The present search report has been drawn up for all claims

1

| Place of search | Date of completion of the search | Examiner |
|---|---|---|
| The Hague | 14 February 2017 | Weiss, Marie-France |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 292 862 A1

## ANNEX TO THE EUROPEAN SEARCH REPORT
## ON EUROPEAN PATENT APPLICATION NO.

EP 16 18 7705

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2017

| Patent document cited in search report | | Publication date | Patent family member(s) | | Publication date |
|---|---|---|---|---|---|
| WO 2010041276 | A1 | 15-04-2010 | EP | 2331084 A1 | 15-06-2011 |
| | | | WO | 2010041276 A1 | 15-04-2010 |
| EP 0519870 | A1 | 23-12-1992 | AT | 142490 T | 15-09-1996 |
| | | | CA | 2071319 A1 | 18-12-1992 |
| | | | DE | 59207094 D1 | 17-10-1996 |
| | | | DK | 0519870 T3 | 13-01-1997 |
| | | | EP | 0519870 A1 | 23-12-1992 |
| | | | EP | 0520119 A1 | 30-12-1992 |
| | | | US | 5711967 A | 27-01-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0005129 A
- EP 0247983 A
- WO 9325204 A
- WO 98052564 A
- WO 9623500 A
- EP 1108425 A
- WO 04014345 A
- EP 0277741 A

**Non-patent literature cited in the description**

- **PILBRANT ; CEDERBERG.** *Scand. J. Gastroenterology,* 1985, vol. 20 (108), 113-120
- European Pharmacopoeia. 2011
- Eudragit® Application Guidelines. June 2008
- Sieve analysis. European Pharmaocopeia. 2011
- European Pharmaocopeia. 2011